(19) Europäisches Patentamt
European Patent Office
Office européen des brevets

(11) Veröffentlichungsnummer : **0 017 121**
**B1**

(12) # EUROPÄISCHE PATENTSCHRIFT

(45) Veröffentlichungstag der Patentschrift : **20.04.83**

(51) Int. Cl.³ : **C 08 G 77/38**, A 61 K 7/06, C 07 F 7/08

(21) Anmeldenummer : **80101535.5**

(22) Anmeldetag : **24.03.80**

(54) **Neue quartäre Polysiloxanderivate, deren Verwendung in Haarkosmetika, sowie diese enthaltende Haarwasch- und Haarbehandlungsmittel.**

(30) Priorität : **29.03.79 DE 2912485**

(43) Veröffentlichungstag der Anmeldung : **15.10.80 Patentblatt 80/21**

(45) Bekanntmachung des Hinweises auf die Patenterteilung : **20.04.83 Patentblatt 83/16**

(84) Benannte Vertragsstaaten : **AT BE CH DE FR GB IT NL SE**

(56) Entgegenhaltungen :
**US A 3 389 160**
**US A 4 005 117**
**US A 4 006 176**
**US A 4 185 087**

**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf 1 (DE)**

(72) Erfinder : **Petzold, Manfred**
**Am Falder 93**
**D-4000 Düsseldorf 13 (DE)**
Erfinder : **Uphues, Günther**
**Himmelgeister Strasse 143**
**D-4000 Düsseldorf (DE)**
Erfinder : **Hempel, Hans Ulrich, Dr.**
**Wiesengrund 5**
**D-5063 Overath/Vilkerath (DE)**
Erfinder : **Scheuermann, Fanny, Dr. geb. Esano-Solomon**
**Volmerswerther Strasse 66**
**D-4000 Düsseldorf (DE)**

**EP 0 017 121 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

« Neue quartäre Polysiloxanderivate, deren Verwendung in Haarkosmetika, sowie diese enthaltende Haarwasch- und Haarbehandlungsmittel »

Die Erfindung betrifft neue quartäre Polysiloxan-Ammonium-Derivate, deren Verwendung in Haarkosmetika zur Verbesserung der Kämmbarkeit, Weichheit und Fülle, sowie diese enthaltende Haarwasch- und Haarbehandlungsmittel.

Es wurde gefunden, daß quartäre Polysiloxan-Ammonium-Derivate der allgemeinen Formel

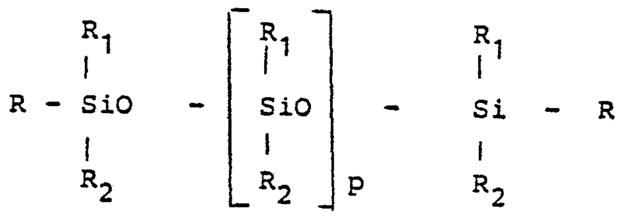

in der $R_1$ und $R_2$ einen Alkylrest mit 1-4 Kohlenstoffatomen oder einen Arylrest, p die Zahlen 0 bis 50, vorzugsweise 10 bis 20 und R die Reste

a)

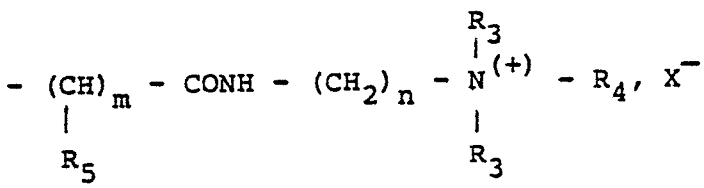

oder

b)

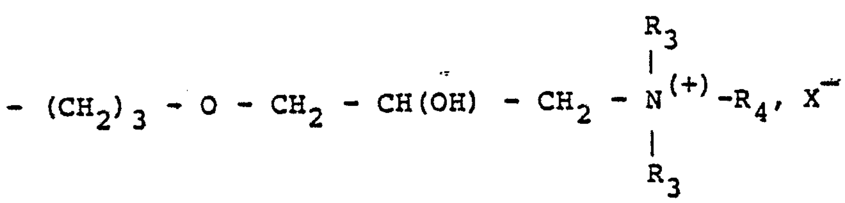

darstellen, wobei $R_3$ einen Alkyl- oder Hydroxyalkylrest mit 1-3 Kohlenstoffatomen, $R_4$ einen Rest gleich $R_3$ oder Aryl—$CH_2$— oder den Allylrest, $R_5$ Wasserstoff oder den Methylrest, $X^-$ die Anionen $Cl^-$, $Br^-$, $J^-$, $CH_3SO_4^-$ oder $C_2H_5SO_4^-$ und m die Zahlen 2-10, vorzugsweise 10, n die Zahlen 2-4, vorzugsweise 3, bedeuten in vorteilhafter Weise für die Herstellung von Haarkosmetika geeignet sind. Haarwasch- und Haarbehandlungsmittel, die diese quartären Polysiloxan-Ammonium-Derivate enthalten, verleihen den damit behandelten Haaren eine ausgezeichnete Kämmbarkeit, sowie Weichheit und Fülle.

Die Herstellung der erfindungsgemäßen Polysiloxan-Ammonium-Derivate erfolgt nach bekannten Verfahren der organischen Chemie. Gemäß einer Herstellungsweise werden an Polydialkylsiloxane mit Silanwasserstoffgruppen in Gegenwart von Platin auf Trägermaterial oder in Gegenwart von Platinchlorwasserstoffsäure ungesättigte Carbonsäureester unter üblichen Bedingungen angelagert. Die erhaltenen Polysiloxanalkylencarbonsäureester werden mit Aminoalkylentertiäraminen umgesetzt; das erhaltene Amidotertiäramin wird anschließend mit üblichen Alkylierungsmitteln wie z. B. Benzylchlorid, Methylchlorid, Dimethylsulfat, gegebenenfalls in Gegenwart von Lösungsmitteln, quaterniert. Die Reaktion verläuft dabei nach folgendem Schema:

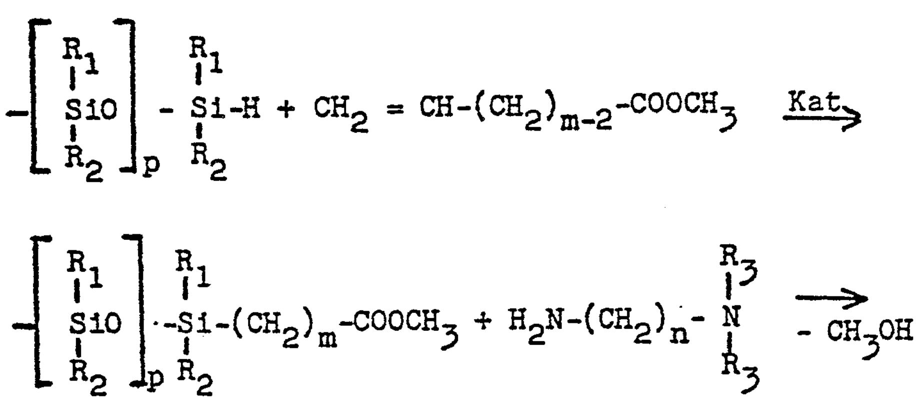

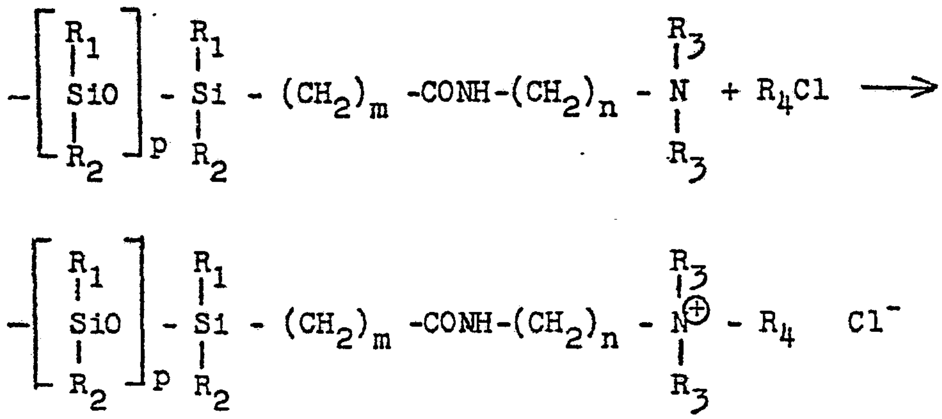

Das vorstehende Schema betrifft Verbindungen mit Resten R der unter a) angegebenen Struktur. Die Herstellung von Verbindungen mit Resten R der unter b) genannten Struktur erfolgt nach bekannten Verfahren gemäß folgendem Schema :

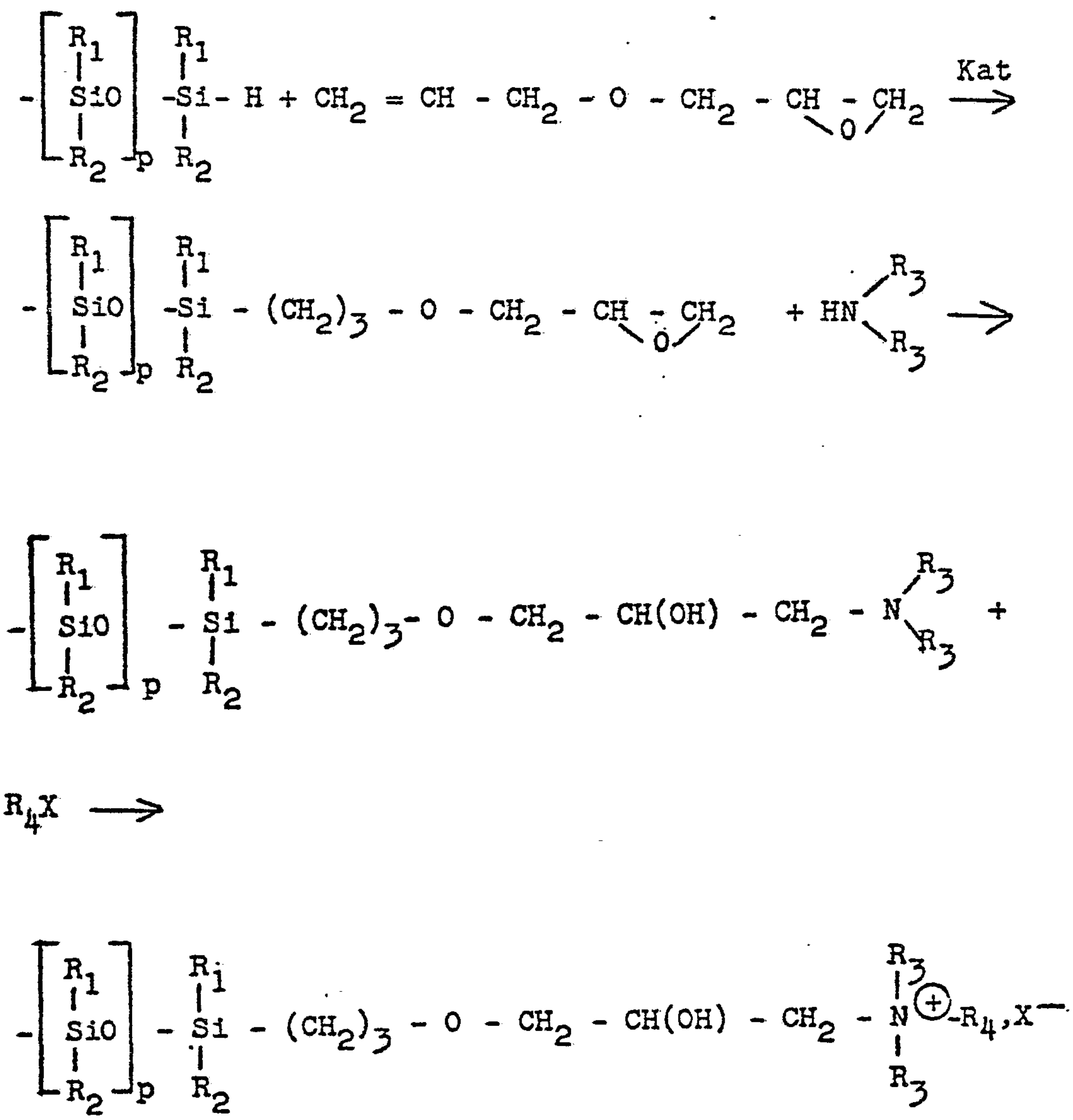


Nach einem anderen Herstellungsverfahren geht man von Chlorsilanen beziehungsweise entsprechenden Methoxysilanen aus, wobei die Reaktion gemäß folgendem Schema durchgeführt wird :

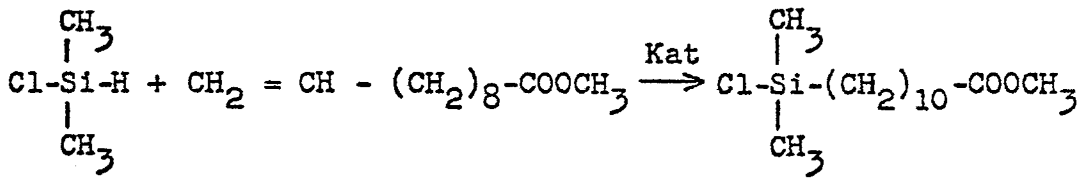


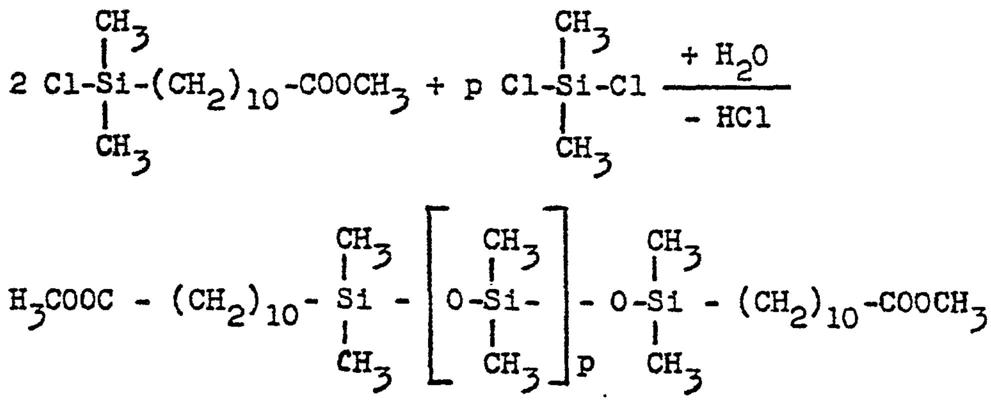


Die weitere Umsetzung zu dem erfindungsgemäßen Polysiloxan-Ammoniumderivat erfolgt dann entsprechend dem vorgenannten Schema.

Die Herstellung der γ-Propylglycidylethergruppen enthaltenden Polysiloxane gestaltet sich entsprechend den Angaben der deutschen Auslegeschrift 13 00 290 in folgender Weise : Ein durch Hydrolyse eines Gemisches aus Dimethyldichlorsilan und Dimethylmonochlorsilan mit Wasser im Überschuß erhaltenes Öl wird in Gegenwart eines Katalysators (Platin auf Aluminiumoxid) bei einer Temperatur um 150 °C mit Allylglycidylether umgesetzt. Die Reaktion verläuft dabei nach folgendem Schema :

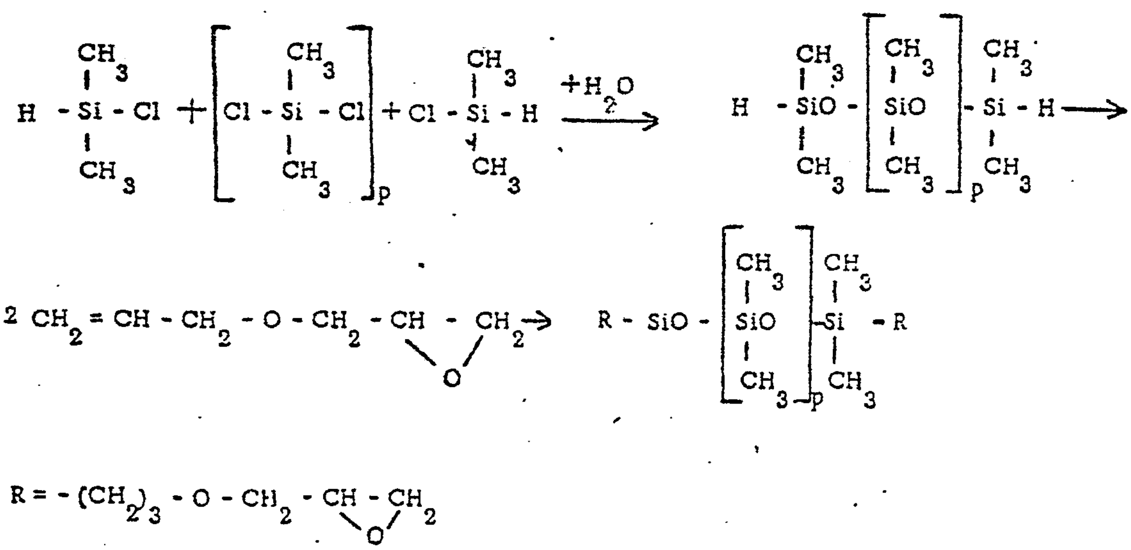


Die weitere Umsetzung mit Dialkylamin und Quaternierungsmittel zu dem erfindungsgemäßen Polysiloxan-Ammonium-Derivat erfolgt dann entsprechend dem vorgenannten Schema.

Als Reaktionskomponente für die Umsetzung der Polydialkylsiloxane beziehungsweise Chlorsilane zu den entsprechenden Carbonsäureestern können alle eine endständige Doppelbindung tragenden aliphatischen Carbonsäuremethylester dienen, wie zum Beispiel Methylester der Acrylsäure, Methacrylsäure, Undecylensäure. Besondere Bedeutung kommt dabei, sowohl von der leichten Weiterverarbeitbarkeit als auch von der besonders guten kosmetischen Wirkung des Endproduktes her, dem Undecylensäuremethylester zu.

Nach Herstellung der entsprechenden Polysiloxan-Allylglycidylether-Addukte werden diese mit Dialkylaminen beziehungsweise Dihydroxyalkylaminen zu den entsprechenden Tertiäraminderivaten umgesetzt. Als bevorzugter Reaktionspartner dient dabei Dimethylamin.

Als Reaktionskomponente für die Aminolyse der Polysiloxanalkylencarbonsäureester zu den entsprechenden Amidoaminen können N,N-Dialkylaminoalkylamine eingesetzt werden, deren Alkyl- beziehungsweise Hydroxyalkylsubstituenten 1-3 Kohlenstoffatome besitzen und deren Kohlenstoffatomzahl in der Alkylkette 2-4, vorzugsweise 3 beträgt, wie zum Beispiel Dimethylamino-ethylamin, -propylamin,

-butylamin, Diethylamino-ethylamin, -propylamin, -butylamin, Dipropylamino-ethylamin, -propylamin, -butylamin, Di-2-hydroxy-ethylamino-ethylamin, -propylamin, -butylamin, Dibutylaminopropylamin. Unter diesen kommt dem Dimethylaminopropylamin und Diethylaminopropylamin besondere Bedeutung zu.

Für die Quaternierungsreaktion lassen sich als Alkylierungsmittel alle für diesen Zweck gebräuchlichen Verbindungen einsetzen, wie zum Beispiel Methylchlorid, Allylchlorid, Benzylchlorid, Dimethylsulfat, Diethylsulfat, Methylbromid, Methyljodid, Benzylbromid. Für die Durchführbarkeit der Quaternierungsreaktion ist in fast allen Fällen die Mitverwendung von Alkohol als Lösungsmittel erforderlich, da bei nur wässriger Verdünnung gelartige, nicht zu rührende Produkte anfallen. Als bevorzugtes Quaternierungsmittel ist Benzylchlorid anzusehen. Die erfindungsgemäßen Polysiloxan-Ammonium-Derivate selbst stellen flüssige oder feste Substanzen dar, die je nach Größe der Polysiloxankette in Wasser dispergierbar oder löslich sind. In vielen Fällen werden die Reaktionsprodukte als klare bis opaleszente, viskose, ca. 50 %ige Lösungen erhalten.

Bevorzugte Verbindungen unter den erfindungemäßen Polysiloxan-Ammonium-Derivaten stellen demnach Produkte folgender allgemeiner Formel dar:

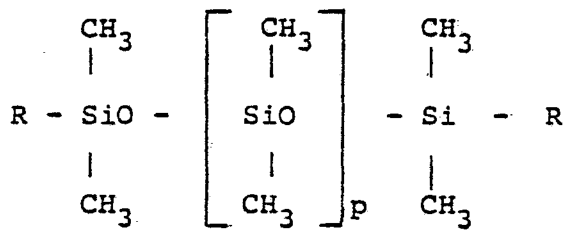

in der R die Reste

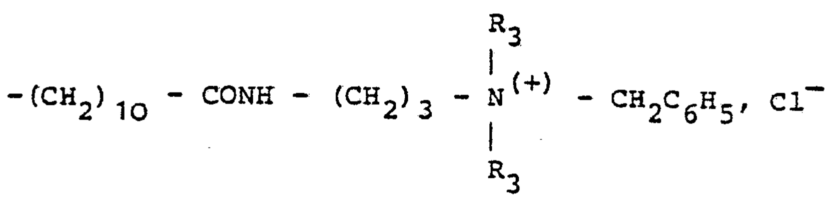

beziehungsweise

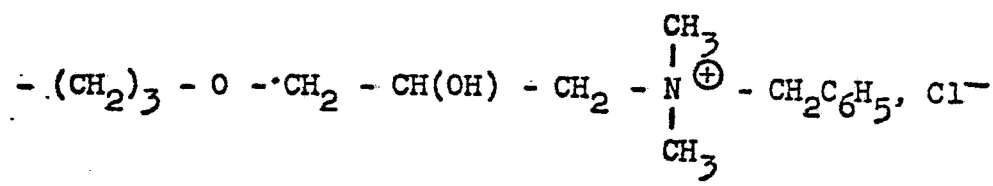

$R_3$ den Methyl- oder Ethylrest und p die Zahlen 10-20 bedeuten.

Eine Isolierung der erfindungsgemäßen Polysiloxan-Ammonium-Derivate in reiner Form ist für den Einsatz in den kosmetischen Präparationen nicht erforderlich, da die Einarbeitung in die Haarwasch- und Haarbehandlungsmittel in Gestalt der viskosen wässrigen Lösung erfolgen kann. Aus praktischen Gründen sollte die Konzentration der wässrigen Lösung nicht wesentlich unter 50 Gewichtsprozent liegen.

Die Einarbeitung der wässrigen Lösungen der Polysiloxan-Ammonium-Derivate in die Haarwasch- und Haarbehandlungsmittel erfolgt in üblicher Weise durch einfaches Einrühren. Dabei bewegt sich die zugesetzte Menge in den Grenzen von 0,1 bis 10 Gewichtsprozent, vorzugsweise 0,5-5 Gewichtsprozent, berechnet als reines Polysiloxan-Ammonium-Derivat und bezogen auf das gesamte haarkosmetische Mittel.

In die erfindungsgemäßen Haarwasch- und Haarbehandlungsmittel können neben dem Polysiloxan-Ammonium-Derivat alle üblichen Zusatzstoffe, wie Proteinhydrolysate, Kräuterauszüge, Rückfettungsmittel, Schaumstabilisatoren, Antischuppenmittel, Parfümöle, Konservierungsmittel, haarfestigende Kunstharze und andere in den für den jeweiligen Verwendungszweck gebräuchlichen Mengen eingearbeitet werden. Aber auch bei dem Einsatz oberflächenaktiver Verbindungen bestehen uneingeschränkte Kombinationsmöglichkeiten, da die erfindungsgemäß einzusetzenden Polysiloxan-Ammonium-Derivate sowohl mit kationaktiven und nichtionogenen Tensiden als auch mit anionaktiven Tensiden sehr gut verträglich sind. Hierdurch wird die Möglichkeit geschaffen, einen sehr gut avivierenden Wirkstoff in Shampoos auf Basis anionaktiver Tenside, wie zum Beispiel Alkylsulfaten, Alkyläthersulfaten einzuarbeiten. Bei diesem Einsatz in erfindungsgemäßen Haarwaschmitteln wird durch die Polysiloxan-Ammonium-Derivate weder die Viskosität noch das Schaumverhalten oder die Waschkraft des Shampoos beeinträchtigt.

Als Beispiele für haarkosmetische Zubereitungen, denen durch den Zusatz der Polysiloxan-Ammonium-Derivate vorteilhafte Eigenschaften verliehen werden können, sind Shampoos, Haarfestiger, Haarkuren, Haarregeneratoren, Haarsprays, Kurspülungen, Haarwässer zu nennen.

Die erfindungsgemäßen Haarwasch- und Haarbehandlungsmittel verleihen dem damit behandelten Haar im Hinblick auf die üblichen Pflegemaßnahmen außergewöhnlich günstige Eigenschaften. So wird die Naßkämmbarkeit der Haare wesentlich verbessert, das trockene Haar ist weich und angenehm im Griff und von guter Fülle und läßt sich ohne Schwierigkeiten frisieren. Die statische Aufladung des trockenen Haares ist weitgehend herabgesetzt und das behandelte Haar behält die frisiertechnisch günstigen Eigenschaften über einen längeren Zeitraum bei. Über die wesentliche Verbesserung der frisiertechnischen Eigenschaften des Haares hinaus, bewirkt die Behandlung mit den erfindungsgemäßen Haarwasch- und Haarbehandlungsmitteln eine erhebliche Steigerung des Glanzes der behandelten Haare. Weiterhin ist die besonders gute physiologische Verträglichkeit der einzusetzenden Produkte zu erwähnen.

Polysiloxanverbindungen mit quartäre Ammoniumgruppen tragenden Seitenketten werden in der US-PS 3.389.160 beschrieben. Von diesen Verbindungen ist bekannt, daß sie starke oberflächenaktive Wirkung haben und als Korrosionsinhibitoren für wässrige Medien geeignet sind. Über eine Anwendung dieser oder ähnlicher Verbindungen als haarkosmetische Wirkstoffe ist dieser Druckschrift nichts zu entnehmen.

Demgegenüber weisen die erfindungsgemäßen Polysiloxanderivate nur jeweils zwei quartäre Ammoniumgruppen in Form von Endgruppen auf. Überraschend zeigen die erfindungsgemäßen Polysiloxanderivate bei der Anwendung in haarkosmetischen Mitteln anwendungstechnische Vorteile, vor allem bei der Verbesserung der Naßkämmbarkeit des damit behandelten Haares.

Die nachfolgenden Beispiele sollen den Gegenstand der Erfindung näher erläutern, ohne ihn jedoch hierauf zu beschränken.

## Beispiele

Zunächst wird die Herstellung der erfindungsgemäßen Polysiloxan-Ammonium-Derivate beschrieben.

$A_1$) Herstellung der Dimethylchlorsilan-Undecylensäureester-Addukte :

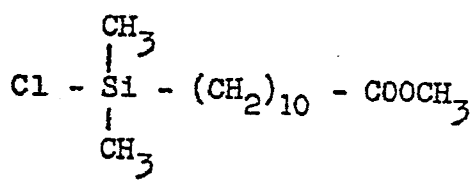

(I)

Die Herstellung erfolgte durch Addition von Undecylensäuremethylester an Dimethylchlorsilan gemäß den Angaben von Nasser Saghian und David Gertner in J. Am. Oil Chemist's Soc. 51 (1974), Seite 363 ff.

$A_2$) Herstellung der Polydimethylsiloxane mit Udecansäuremethylestergruppen in α, ω-Stellung :

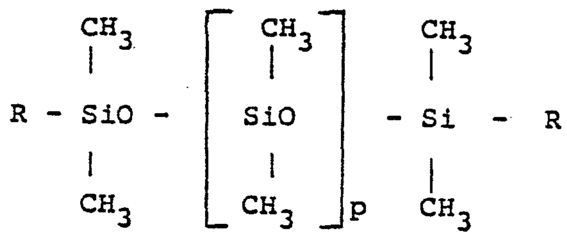

(II)

$R = -(CH_2)_{10}-COOCH_3$ , p ca. 17

Die Herstellung erfolgt durch Cohydrolyse der unter $A_1$ hergestellten Verbindung (1) mit Dimethyldichlorsilan auf folgendem Wege :

293 g (1 Mol) der Verbindung (1) und 1 290 g (10 Mol) Dimethyldichlorsilan wurden gemischt und bei Raumtemperatur (Kühlung mit Eis) in 7 Liter Wasser eingetropft. Nach beendeter Zugabe würde 1 Stunde lang bei Raumtemperatur nachgerührt. Die Siloxanphase wurde abgetrennt, mit Natriumhydrogencarbonat-Lösung und Wasser neutralgewaschen, über Natriumsulfat getrocknet und filtriert. Das erhaltene Rohprodukt wurde mit 3 % Bleicherde (Tonsil LFF80) versetzt und unter Stickstoff 16 Stunden

lang auf 120 °C erhitzt. Danach wurde die Bleicherde abfiltriert und das Filtrat zur Entfernung leichtflüchtiger Anteile bis 180 °C/18 mbar andestilliert. Es entstand ein klares Öl mit einer Viskosität von 39 m Pa.s (20 °C), einer Verseifungszahl von 62,4 und einem Molgewicht von 1 800.

In völlig analoger Weise wurden weitere Polydimethylsiloxane mit Undecansäuremethylestergruppen in α, ω-Stellung hergestellt, die in nachstehender Tabelle 1 aufgeführt sind :

Tabelle 1

| Verbindung | Einsatzkomponenten | | Kenndaten | | | |
|---|---|---|---|---|---|---|
| | (2) in g | Dimethyl-dichlor-silan in g | p ca. | VZ | Visk. m Pa.s | $\overline{MG}$ Ber. |
| $B_2$ | 585,0 | 1 935,0 | 13 | 75,3 | 34,0 | 1 500 |
| $C_2$ | 351,0 | 1 935,0 | 20,3 | 55,3 | 44,0 | 2 000 |
| $D_2$ | 219 | 1 935,0 | 32 | 38,6 | 67 | 2 900 |

$G_1$) Herstellung der Polysiloxane mit γ-Propylglycidylethergruppen.

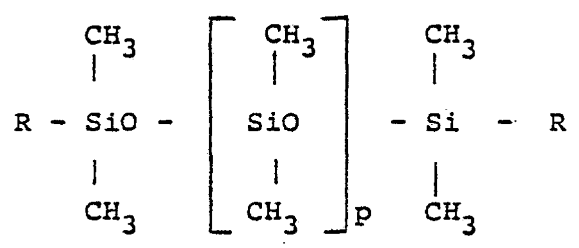

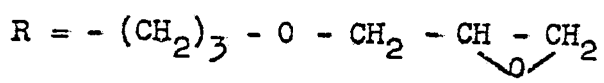

Zur Herstellung der Verbindung wurden 114 g Allylglycidylether (1 Mol) und 2 g eines mit 5 Gewichtsprozent Platin beladenen Aktivkohle-Katalysators in Stickstoffatmosphäre bei einer Temperatur zwischen 90 und 100 °C unter Rühren mit 516 g (0,45 Mol) eines Polymethyl-H-Siloxans (obige Formel R = H, p ca. 14) versetzt. Der Ansatz wurde bei dieser Temperatur belassen, bis im Infrarotspektrum einer Probe keine Si-H-Banden mehr vorhanden waren (nach circa 4 Stunden). Danach wurde auf Raumtemperatur abgekühlt, filtriert und zur Entfernung überschüssigen Allylglycidylethers im Ölpumpenvakuum bis 130 °C andestilliert.

Ein weiteres Polysiloxan mit γ-Propylglycidylethergruppen wurde in analoger Weise hergestellt. Die nachstehende Tabelle 2 gibt die Kenndaten der beiden Verbindungen $G_1$ und $H_1$ wieder.

Tabelle 2

| Verbindung | p | % Ep-0 | Visk. m Pa.s | $\overline{MG}$ ber. |
|---|---|---|---|---|
| $G_1$ | 14 | 2,28 | 23 | 1 400 |
| $H_1$ | 29 | 1,29 | 43 | 2 500 |

Herstellung der tertiären Aminopolysiloxane durch Aminolyse der Polysiloxan-Undecylensäuremethylester-Addukte. Für die Aminolyse diente Dimethylaminopropylamin (DMAP) beziehungsweise Diethylaminopropylamin (DEAP)

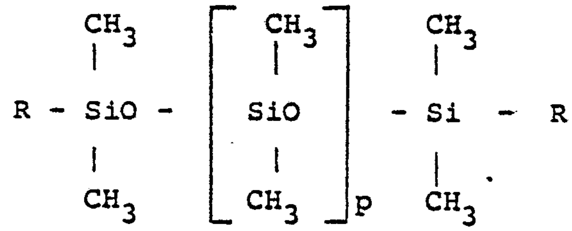 (III)

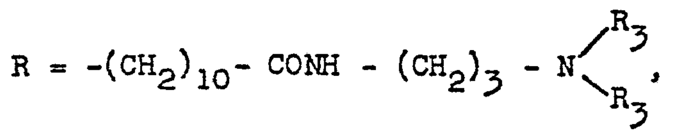 , $R_3 = CH_3$ bzw. $C_2H_5$

Die Herstellung erfolgte durch Aminolyse der unter $A_2$ hergestellten Verbindung (2) mit Dimethylaminopropylamin auf folgendem Wege : 135,0 g (0,15 Äquivalent) des Polysiloxanderivats ($A_2$) und 30,6 g (0,3 Mol) Dimethylaminopropylamin wurden im Autoklaven erwärmt und 2 Stunden lang auf einer Temperatur von 175 °C gehalten. Nach Abkühlung und Entspannung wurde das Reaktionsprodukt in eine Destillationsapparatur überführt. Im Wasserstrahlvakuum wurden dann bis zu einer Temperatur von 140 °C das gebildete Methanol und nicht umgesetztes Dimethylaminopropylamin entfernt. Es wurden 144,1 g einer viskosen Flüssigkeit erhalten, deren Analyse 1,46 % Aminstickstoff ergab. Das Produkt war in verdünnter Essigsäure klar löslich. Das Molgewicht theoretisch beträgt 1944.

In analoger Weise wurden folgende tertiäre Aminopolysiloxane hergestellt, die in nachstehender Tabelle 3 aufgeführt sind.

Tabelle 3

| Verbindung | Ausgangs-Polysiloxanderivate | Aminkomp. | Molgewicht theoretisch | % N-Amin | lösl. in verdünnter Essigsäure |
|---|---|---|---|---|---|
| $B_3$ | $B_2$ | DMAP | 1 640 | 1,7 | klar |
| $C_3$ | $C_2$ | DMAP | 2 140 | 1,3 | fast klar |
| $D_3$ | $D_2$ | DMAP | 3 040 | 0,9 | trüb |
| $E_3$ | $A_2$ | DEAP | 2 000 | 1,49 | fast klar |

$G_2$) Herstellung der tertiären Aminopolysiloxane durch Addition von Aminen an Polysiloxane mit γ-Propylglycidylethergruppen. Für die Umsetzung diente Dimethylamin.

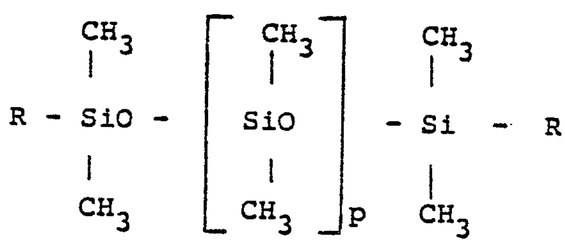

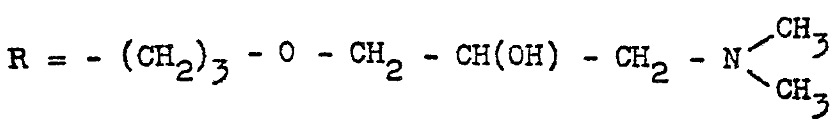

Die Herstellung erfolgte durch Erhitzen der Reaktionskomponenten im Autoklaven auf 120 °C. Um zu besseren Ausbeuten zu kommen, wurde das Amin in Form einer 40 %igen wässrigen Lösung im Überschuß eingesetzt. Nach beendigter Reaktion wurde das überschüssige Amin sowie das Wasser abdestilliert. Die Reaktionsprodukte stellen viskose Flüssigkeiten dar, deren weitere Werte der Tabelle 4 zu entnehmen sind.

Tabelle 4

| Verbindung | Ausgangs-polysiloxan | Molgewicht theor. | % N-Amin | Löslich in verdünnter Essigsäure |
|---|---|---|---|---|
| $G_2$ | $G_1$ | 1 490 | 1,95 | etwas trüb |
| $H_2$ | $H_1$ | 2 590 | 1,3 | trüb, homogen |

$A_4$) Herstellung der erfindungsgemäßen quartären Polysiloxan-Ammonium-Derivate durch Umsetzung der tertiären Aminopolysiloxane mit Benzylchlorid beziehungsweise anderen Quaternierungsmitteln.

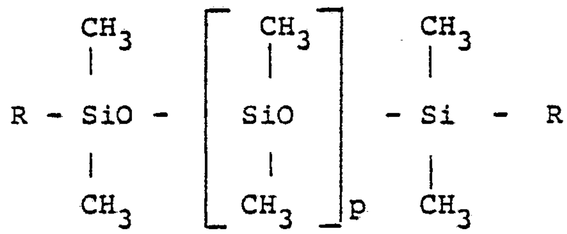

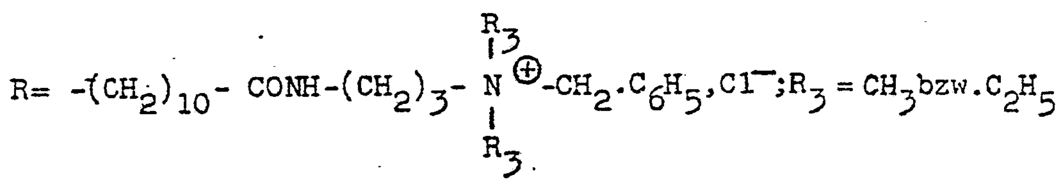

beziehungsweise

$R = -(CH_2)_3-O-CH_2-CH(OH)-CH_2-N^{\oplus}(CH_3)_2-CH_2-C_6H_5,\ Cl^-$

Die Herstellung erfolgte durch Umsetzung des unter $A_3$ genannten tertiären Aminopolysiloxans (3) mit Benzylchlorid auf folgendem Wege:

60,0 g (0,06 Äquivalent) des Polysiloxan-undecansäureamidoamins ($A_3$) wurden mit 29,0 g Isopropanol und 38,2 g Wasser gemischt. Zu der inhomogenen Flüssigkeit wurden dann bei 80 °C 7,2 g (0,057 Mol) Benzylchlorid getropft. Schon kurz nach Beginn des Zutropfens bildete sich eine klare Lösung. Bei 80 °C wurde noch 2 Stunden lang nachgerührt. Das Reaktionsprodukt bildet eine klare, ölige Flüssigkeit, die bei der Verdünnung mit Wasser fast klare Lösungen ergibt.

In analoger Weise wurden noch folgende erfindungsgemäße Polysiloxan-Ammonium-Derivate hergestellt, die in Tabelle 5 aufgeführt sind.

Tabelle 5

| Verbindung | tert. Aminopolysiloxan Ausgangsprodukt | Quaternierungsmittel | Wasserlöslichkeit |
|---|---|---|---|
| $B_4$ | $B_3$ | Benzylchlorid | klar |
| $C_4$ | $C_3$ | Benzylchlorid | fast klar |
| $D_4$ | $D_3$ | Benzylchlorid | trüb |
| $E_4$ | $E_3$ | Benzylchlorid | fast klar |
| $F_4$ | $A_3$ | Methylchlorid | klar |
| $G_3$ | $G_2$ | Benzylchlorid | trüb |
| $H_3$ | $H_2$ | Benzylchlorid | zum Teil unlöslich |

Akute Toxizität

Die Verbindung $A_4$ der vorgenannten Formel in der $R_3 = CH_3$ ist, wurde auf ihre Verträglichkeit an erwachsenen männlichen Wistar-Ratten geprüft. Die Verabfolgung der Prüfsubstanz erfolgte einmal in steigender Dosierung mit der Schlundsonde. Es wurden pro Dosis 10 Versuchstiere eingesetzt. Dabei ergab sich folgende akute Toxizität:

Ratte oral $LD_{50} > 10$ g/kg als 100 %iges Produkt.

Nachstehend werden einige Rezepturen von Haarwasch- und Haarbehandlungsmitteln mit einem Gehalt an den erfindungsgemäßen Polysiloxan-Ammonium-Derivaten aufgeführt.

Shampoo

| | |
|---|---|
| Natriumlauryläthersulfat mit 27-28 % Waschaktivsubstanz | 30,0 Gewichtsteile |
| Natriumchlorid | 2,0 Gewichtsteile |
| Kokosfettsäurediäthanolamid erfindungsgemäße quartäre | 2,0 Gewichtsteile |
| Polysiloxan-Ammonium-Verbindungen $A_4$ | 5,0 Gewichtsteile (als 50 %ige wäßrige Lösung) |
| Zinkpyridinthion | 1,0 Gewichtsteile |
| Proteinhydrolysat | 1,0 Gewichtsteile |
| Duftkomponente | 1,0 Gewichtsteile |
| Wasser | 58,0 Gewichtsteile |

Shampoo

| | |
|---|---|
| Natriumlauryläthersulfat mit 35-37 % Waschaktivubstanz | 30,0 Gewichtsteile |
| Natriumchlorid erfindungsgemäße quartäre | 1,0 Gewichtsteile |
| Polysiloxan-Ammonium-Verbindungen $B_4$ | 8,0 Gewichtsteile (als 50 %ige wäßrige Lösung) |
| Duftkomponente | 1,0 Gewichtsteile |
| Wasser | 60,0 Gewichtsteile |

Shampoo für fettes Haar

| | |
|---|---|
| Magnesiumlauryläthersulfat 29-31 % Waschaktivsubstanz | 30,0 Gewichtsteile |
| Kokosfettsäurediäthanolamid erfindungsgemäße quartäre | 5,0 Gewichtsteile |
| Polysiloxan-Ammonium-Verbindungen $C_4$ | 5,0 Gewichtsteile (als 50 %ige wäßrige Lösung) |
| Glycerin-$C_{8-18}$-fettsäureester + 7 Mol Äthylenoxid | 3,0 Gewichtsteile |
| Proteinhydrolysat | 1,0 Gewichtsteile |
| p-Hydroxybenzoesäuremethylester | 0,2 Gewichtsteile |
| Duftkomponente | 1,0 Gewichtsteile |
| Wasser | 54,8 Gewichtsteile |

Haarfestiger

| | |
|---|---|
| Mischpolymeres aus Polyvinylpyrrolidon/ Polyvinylacetat 30:70 erfindungsgemäße quartäre | 2,0 Gewichtsteile |
| Polysiloxan-Ammonium-Verbindung $D_4$ | 3,0 Gewichtsteile (als 50 %ige wäßrige Lösung) |
| Polyglykol | 0,2 Gewichtsteile |
| Äthanol | 34,8 Gewichtsteile |
| Parfümöl | 1,0 Gewichtsteile |
| Wasser | 59,0 Gewichtsteile |

Haarkurspülung

| | |
|---|---|
| Cetylalkohol | 3,0 Gewichtsteile |
| Vaselinöl erfindungsgemäße quartäre | 2,0 Gewichtsteile |
| Polysiloxan-Ammonium-Verbindung $E_4$ | 5,0 Gewichtsteile (als 50 %ige wäßrige Lösung) |
| $C_{8-18}$-Alkyl-dimethyl-aceto-betain | 5,0 Gewichtsteile |
| Zitronensäure | 1,0 Gewichtsteile |
| Kräuterauszüge | 1,0 Gewichtsteile |
| Parfümöl | 1,0 Gewichtsteile |
| Wasser | 82,0 Gewichtsteile |

Haarregenerator

| | |
|---|---|
| Cetylstearylalkohol | 4,0 Gewichtsteile |
| Zitronensäure | 1,0 Gewichtsteile |
| Parfüm, wasserlöslich erfindungsgemäße quartäre | 1,0 Gewichtsteile |

| | |
|---|---|
| Polysiloxan-Ammonium-Verbindung $F_4$ | 3,0 Gewichtsteile (als 50 %ige wäßrige Lösung) |
| Wasser | 91,0 Gewichtsteile |

Schnellhaarkur

| | |
|---|---|
| Cetylalkohol erfindungsgemäße quartäre | 6,0 Gewichtsteile |
| Polysiloxan-Ammonium-Verbindung $A_4$ | 10,0 Gewichtsteile (als 50 %ige wäßrige Lösung) |
| Ölsäuredecylester $C_{8-18}$-Alkyl-dimethyl-aceto-betain | 5,0 Gewichtsteile |
| Zitronensäure | 1,0 Gewichtsteile |
| Kräuterauszüge | 1,0 Gewichtsteile |
| Parfümöl | 1,0 Gewichtsteile |
| Wasser | 73,0 Gewichtsteile |

Haarspray

| | |
|---|---|
| Mischpolymeres aus Polyvinyl-pyrrolidon/ | |
| Polyvinylacetat 60 : 40 erfindungsgemäße quartäre | 4,0 Gewichtsteile |
| Polysiloxan-Ammonium-Verbindung $B_4$ | 2,0 Gewichtsteile (als 50 %ige wäßrige Lösung) |
| Duftkomponente | 2,0 Gewichtsteile |
| Äthanol 96 %ig | 92,0 Gewichtsteile |

Abfüllung : 40 % Haarlack obiger Rezeptur
60 % Treibgas (Difluordichlormethan-Monofluor-trichlormethan 50 : 50)

Haarwasser gegen Schuppen

| | |
|---|---|
| Oleyl-Cetylalkohol | 0,5 Gewichtsteile |
| Isopropanol | 62,0 Gewichtsteile |
| Kräuterauszüge | 4,0 Gewichtsteile |
| Menthol | 0,2 Gewichtsteile |
| Proteinhydrolysat | 1,0 Gewichtsteile |
| pantothensaures Calcium | 0,05 Gewichtsteile |
| Vitamin H erfindungsgemäße quartäre | 0,30 Gewichtsteile |
| Polysiloxan-Ammonium-Verbindung $G_3$ | 6,0 Gewichtsteile (als 50 %ige wäßrige Lösung) |
| Parfümöl | 1,0 Gewichtsteile |
| Wasser | 25,0 Gewichtsteile |

**Ansprüche**

1. Polysiloxan-Ammonium-Derivate der allgemeinen Formel

$$R - \overset{\displaystyle R_1}{\underset{\displaystyle R_2}{\overset{|}{\underset{|}{SiO}}}} - \left[ \overset{\displaystyle R_1}{\underset{\displaystyle R_2}{\overset{|}{\underset{|}{SiO}}}} \right]_p - \overset{\displaystyle R_1}{\underset{\displaystyle R_2}{\overset{|}{\underset{|}{Si}}}} - R$$

in der $R_1$ und $R_2$ einen Alkylrest mit 1-4 Kohlenstoffatomen oder einen Arylrest, p die Zahlen 0 bis 50 und R die Reste

a) $$- \underset{\displaystyle R_5}{\underset{|}{(CH)}}_m - CONH - (CH_2)_n - \overset{\displaystyle R_3}{\underset{\displaystyle R_3}{\overset{|}{\underset{|}{N}}}}{}^{(+)} - R_4,\ X^-$$

oder

b) $$- (CH_2)_3 - O - CH_2 - CH(OH) - CH_2 - \overset{\displaystyle R_3}{\underset{\displaystyle R_3}{\overset{|}{\underset{|}{N}}}}{}^{(+)} - R_4,\ X^-$$

darstellen, wobei $R_3$ einen Alkyl- oder Hydroxy-alkylrest mit 1-3 Kohlenstoffatomen, $R_4$ einen Rest gleich $R_3$ oder Aryl—$CH_2$— oder den Allylrest, $R_5$ Wasserstoff oder den Methylrest, $X^-$ die Anionen $Cl^-$, $Br^-$, $J^-$, $CH_3SO_4^-$ oder $C_2H_5SO_4^-$ und m die Zahlen 2-10, n die Zahlen 2-4, bedeuten.

2. Polysiloxan-Ammonium-Derivate nach Anspruch 1, wobei in der allgemeinen Formel p die Zahlen 10 bis 20, m die Zahl 10 und n die Zahl 3 bedeutet.

3. Polysiloxan-Ammonium-Derivate nach Anspruch 1 der Formel

$$R-\underset{CH_3}{\overset{CH_3}{\underset{|}{\overset{|}{SiO}}}}-\left[\underset{CH_3}{\overset{CH_3}{\underset{|}{\overset{|}{SiO}}}}\right]_p-\underset{CH_3}{\overset{CH_3}{\underset{|}{\overset{|}{Si}}}}-R$$

in der p die Zahlen 10 bis 20 und R die Reste

a) $$-(CH_2)_{10}-CONH-(CH_2)_3-\underset{R_3}{\overset{R_3}{\underset{|}{\overset{|}{N^{(+)}}}}}-CH_2C_6H_5,\ Cl^-$$

oder

b) $$-(CH_2)_3-O-CH_2-CH(OH)-CH_2-N^{(+)}(CH_3)_2-CH_2C_6H_5, Cl^-$$

bedeuten, wobei $R_3$ den Methyl- oder Ethylrest darstellt.

4. Verwendung der Polysiloxan-Ammonium-Derivate nach Anspruch 1 bis 3, in Haarwasch- und Haarbehandlungsmitteln zur Verbesserung der frisiertechnischen Eigenschaften der Haare.

5. Haarwasch- und Haarbehandlungsmittel, gekennzeichnet durch einen Gehalt an Polysiloxan-Ammonium-Derivaten nach Anspruch 1 bis 3 in einer Menge von 0,1 bis 10 Gew.-%, berechnet als reine Verbindung und bezogen auf das gesamte haarkosmetische Mittel und einem nichttoxischen Träger.

6. Haarwasch- und Haarbehandlungsmittel nach Anspruch 5, dadurch gekennzeichnet, daß sie neben dem Polysiloxan-Ammonium-Derivat andere übliche Wirkstoffe in den für den jeweiligen Verwendungszweck gebräuchlichen Mengen enthalten.

## Claims

1. Polysiloxane-ammonium derivatives corresponding to the following general formula

$$R-\underset{R_2}{\overset{R_1}{\underset{|}{\overset{|}{SiO}}}}-\left[\underset{R_2}{\overset{R_1}{\underset{|}{\overset{|}{SiO}}}}\right]_p-\underset{R_2}{\overset{R_1}{\underset{|}{\overset{|}{Si}}}}-R$$

in which $R_1$ and $R_2$ represent a $C_1$-$C_4$ alkyl radical or an aryl radical, p is a number of from 0 to 50 and R represents the radicals

a) $$-\underset{R_5}{\underset{|}{(CH)_m}}-CONH-(CH_2)_n-\underset{R_3}{\overset{R_3}{\underset{|}{\overset{|}{N^{(+)}}}}}-R_4,\ X^-$$

or

b) $$-(CH_2)_3-O-CH_2-CH(OH)-CH_2-\overset{\displaystyle R_3}{\underset{\displaystyle R_3}{N^{(+)}}}-R_4,\ X^-$$

wherein $R_3$ represents a $C_1$-$C_3$ alkyl or hydroxyalkyl radical, $R_4$ has the same meaning as $R_3$ or represents aryl—$CH_2$— or the allyl radical, $R_5$ represents hydrogen or the methyl radical, $X^-$ represents the anions $Cl^-$, $Br^-$, $I^-$, $CH_3SO_4^-$ or $C_2H_5SO_4^-$ and m is a number of from 2 to 10 and n a number of from 2 to 4.

2. Polysiloxane-ammonium derivatives as claimed in Claim 1, p in the general formula being a number of from 10 to 20, m the number 10 and n the number 3.

3. Polysiloxane-ammonium derivatives as claimed in Claim 1 corresponding to the following formula

$$R-\overset{\displaystyle CH_3}{\underset{\displaystyle CH_3}{SiO}}-\left[\overset{\displaystyle CH_3}{\underset{\displaystyle CH_3}{SiO}}\right]_p-\overset{\displaystyle CH_3}{\underset{\displaystyle CH_3}{Si}}-R$$

in which p is a number of from 10 to 20 and R represents the radicals

a) $$-(CH_2)_{10}-CONH-(CH_2)_3-\overset{\displaystyle R_3}{\underset{\displaystyle R_3}{N^{(+)}}}-CH_2C_6H_5,\ Cl^-$$

or

b) $$-(CH_2)_3-O-CH_2-CH(OH)-CH_2-N^{(+)}(CH_3)_2-CH_2C_6H_5,\ Cl^-$$

wherein $R_3$ represents the methyl or ethyl radical.

4. The use of the polysiloxane-ammonium derivatives claimed in Claims 1 to 3 in hair-washing and hair-treatment preparations for improving the hairdressing properties of hair.

5. Hair-washing and hair-treatment preparations, characterised by a content of the polysiloxane-ammonium derivatives claimed in Claims 1 to 3 amounting to between 0.1 and 10 % by weight, expressed as pure compound and based on the hair-cosmetic preparation as a whole and a non-toxic vehicle.

6. Hair-washing and hair-treatment preparations as claimed in Claim 5, characterised in that, in addition to the polysiloxane-ammonium derivative, they contain other standard active substances in the usual quantities for the application envisaged.

**Revendications**

1. Dérivés de polysiloxane-ammonium de formule générale

$$R-\overset{\displaystyle R_1}{\underset{\displaystyle R_2}{SiO}}-\left[\overset{\displaystyle R_1}{\underset{\displaystyle R_2}{SiO}}\right]_p-\overset{\displaystyle R_1}{\underset{\displaystyle R_2}{Si}}-R$$

dans laquelle $R_1$ et $R_2$ représentent un reste alkyle en $C_1$-$C_4$ ou un reste aryle, p est un nombre de 0 à 50 et R représente les restes

a)

$$-\underset{\underset{R_5}{|}}{(CH)_m}-CONH-(CH_2)_n-\overset{\overset{R_3}{|}}{\underset{\underset{R_3}{|}}{N^{(+)}}}-R_4,\ X^-$$

ou

b)

$$-(CH_2)_3-O-CH_2-CH(OH)-CH_2-\overset{\overset{R_3}{|}}{\underset{\underset{R_3}{|}}{N^{(+)}}}-R_4,\ X^-$$

dans lesquels $R_3$ représente un reste alkyle ou hydroxyalkyle en $C_1$-$C_3$, $R_4$ représente un reste identique à $R_3$ ou un groupe aryl—$CH_2$— ou le reste allyle, $R_5$ représente l'hydrogène ou le reste méthyle, $X^-$ représente les anions $Cl^-$, $Br^-$, $I^-$, $CH_3SO_4^-$ ou $C_2H_5SO_4^-$ et m est un nombre de 2 à 10 et n un nombre de 2 à 4.

2. Dérivés de polysiloxane-ammonium selon la revendication 1, pour lesquels, dans la formule générale, p est un nombre de 10 à 20, m est égal à 10 et n est égal à 3.

3. Dérivés de polysiloxane-ammonium selon la revendication 1 de formule

$$R-\overset{\overset{CH_3}{|}}{\underset{\underset{CH_3}{|}}{SiO}}-\left[\overset{\overset{CH_3}{|}}{\underset{\underset{CH_3}{|}}{SiO}}\right]_p-\overset{\overset{CH_3}{|}}{\underset{\underset{CH_3}{|}}{Si}}-R$$

dans laquelle p est un nombre de 10 à 20 et R représente les restes

a)

$$-(CH_2)_{10}-CONH-(CH_2)_3-\overset{\overset{R_3}{|}}{\underset{\underset{R_3}{|}}{N^{(+)}}}-CH_2C_6H_5,\ Cl^-\text{oder}$$

ou

b)

$$-(CH_2)_3-O-CH_2-CH(OH)-CH_2-N^{(+)}(CH_3)_2-CH_2C_6H_5,\ Cl^-$$

dans lesquels $R_3$ représente le reste méthyle ou éthyle.

4. Utilisation des dérivés de polysiloxane-ammonium selon les revendications 1 à 3 dans des shampooings et produits de traitement de la chevelure pour l'amélioration des propriétés des cheveux à l'égard des opérations d'ondulation.

5. Shampooings et produits pour le traitement de la chevelure, caractérisés en ce qu'ils contiennent des dérivés de polysiloxane-ammonium selon les revendications 1 à 3 en quantité de 0,1 à 10 % en poids, exprimée en composé pur et rapporté au produit cosmétique capillaire total, et un véhicule non toxique.

6. Shampooings et produits pour le traitement de la chevelure selon la revendication 5, caractérisés en ce qu'ils contiennent, en plus du dérivé de polysiloxane-ammonium, d'autres substances actives usuelles aux proportions habituelles pour le but particulier recherché.